# FASCICULE DE BREVET EUROPEEN

(11) **EP 0 618 965 B1**
(45) Date de publication et mention de la délivrance du brevet: **05.04.2006**
(21) Numéro de dépôt: 93902325.5
(22) Date de dépôt: 11.12.1992
(51) Int. Cl.: C12N 15/10, C12N 15/12, C12N 15/54, C12N 15/85

(54) **PROCEDE DE GENERATION D'UNE DIVERSITE STRUCTURALE ET FONCTIONNELLE DANS UNE SEQUENCE PEPTIDIQUE**
VERFAHREN ZUR BILDUNG EINER STRUKTURELLEN UND FUNKTIONELLEN DIVERSITÄT IN EINER PEPTIDSEQUENZ
METHOD FOR GENERATING STRUCTURAL AND FUNCTIONAL DIVERSITY IN A PEPTIDE SEQUENCE

(30) Priorité: 11.12.1991 FR 9115389
(43) Date de publication de la demande: 12.10.1994
(73) Titulaire: INSTITUT PASTEUR, 75724 Paris Cédex 15 (FR)
(72) Inventeur: KALLENBACH, Sacha, F-92000 Nanterre (FR); DOYEN, Noëlle, F-75005 Paris (FR); ROUGEON, François, F-78120 Poigny-la-Forêt (FR)
(74) Mandataire: Phélip, Bruno
(86) Numéro de dépôt international: PCT/FR1992/001178
(87) Numéro de publication internationale: WO 1993/012228

(56) Documents cités:
- NUCL. ACID RES. vol. 18, no. 22, 25 Novembre 1990, IRL PRESS, OXFORD, ENGLAND; page 6730 S. KALLENBACH ET AL. 'A rapid test for V(D)J recombinase activity' cité dans la demande En entier
- SCIENCE, vol. 248, 22 Juin 1990, AAAS, WASHINGTON, DC, US; pages 1517 - 1523 M.A. OETTINGER ET AL. 'RAG-1 and RAG-2, adjacent genes that synergistically activate V(D)J recombination' cité dans la demande
- SCIENCE vol. 246, 8 Décembre 1989, AAAS, WASHINGTON, DC, US; pages 1275 - 1281 W.D. HUSE ET AL. 'Generation of a large combinatorial library of the immunoglobolin repertoire in phage lambda' cité dans la demande
- CELL vol. 59, 22 Décembre 1989, CELL PRESS, CAMBRIDGE, NA.; pages 1035 - 1048 D.G. SCHATZ ET AL. 'The V(D)J recombination activating gene, RAG-1'
- NUCL. ACID RES. vol. 14, no. 14, 25 Juillet 1986, IRL PRESS, OXFORD, ENGLAND; pages 5777 - 5792 O. KOIWAI ET AL. 'Isolation and characterization of bovine and mouse terminal deoxynecleotidyltransferase cDNAs expressible in mammalian cells'
- MOL. CELL. BIOL. vol. 7, no. 9, Septembre 1987, AM. SOC. MICROBIOL., WASHINGTON, D.C pages 3237 - 3243 N.R. LANDAU ET AL. 'Increased frequency of N-region insertion in a murine pre-B-cell line infected with a terminal deoxynucleotidyl transferase retroviral expression vector'
- PROC. NATL. ACAD SCI. vol. 89, no. 7, 1 Avril 1992, NATL. ACAD SCI., WASHINGTON, DC, US; pages 2799 - 2803 S. KALLENBACH ET AL. 'Three lymphoid-specific factors account for all junctional diversity characteristic of somatic assembly od T-cell receptor and immunoglobulin genes'
- SCHATZ D.G. ET AL: 'The V(D)J recombination activating gene RAG-1' CELL vol. 59, 22 Décembre 1989, pages 1035 - 1048, XP002968444
- 'MEGACLONE I vector' INVITROGEN CATALOG page 23
- LIEBER M.R.: 'The mechanism of V(D)J recombination: a balance of diversity, specificity, and stability' CELL vol. 70, 18 Septembre 1992, pages 873 - 876
- VAN GENT D.C. ET AL: 'The RAG1 and RAG2 proteins establish the 12/23 rule in V(D)J recombination' CELL vol. 85, 05 Avril 1996, pages 107 - 113
- KALLENBACH S. ET AL: 'Three lymphoid-specific factors account for all junctional diversity characteristic of somatic assembly of T-cell receptor and immunoglobulin genes' PROC. NATL. ACAD. SCI. USA vol. 89, Avril 1992, pages 2799 - 2803
- ABBAS A.K. ET AL: 'Cellular and Molecular Immunology', 1991, W. B. SAUNDERS COMPANY, PHILADELPHIA, USA * page 86 - page 87 *

## Description

La présente invention a pour objet un procédé de génération d'une diversité structurale ou fonctionnelle dans une séquence peptidique par introduction d'insertions ou délétions de nucléotides dans la séquence nucléotidique codant pour ladite séquence peptidique .

La présente invention est d'autre part relative à des compositions pharmaceutiques , à des médicaments ou à des réactifs de diagnostic contenant des protéines ou peptides obtenus par ce procédé .

Les gènes matures codant pour les chaînes composant les immunoglobulines et les récepteurs des cellules T sont assemblés précocement lors du développement des lymphocytes à partir de segments de gènes dits de variabilité (V), de soudure , ou de jonction (J), et dans certains cas de diversité (D).

Sept loci sont susceptibles d'être réarrangés par recombinaison de ces fragments .

Les séquences des signaux de recombinaison (RSS) adjacentes à chaque gène fournissent les cibles pour la recombinaison . Ces séquences sont composées d'un heptamère palindromique et d'un nonamère riche en adénosine et en thymidine , séparées par une séquence de 12 ou de 23 paires de base. Les réarrangements s'opèrent entre des RSS avec des séquences de séparation de différentes longueurs .

Deux types de jonction ou soudure sont formés durant la recombinaison : des jonctions codantes créées par la juxtaposition de segments de gènes et des jonctions non codantes formées par des RSS contiguës. Dans ce dernier cas , les heptamères sont généralement joints sans insertions de nucléotide ou sans délétions . Les jonctions codantes sont quant à elles sujettes à des modifications importantes .

Les variations dans les jonctions au cours du réarrangement des segments de gènes codant pour les immunoglobulines constituent une grande source de diversité . Plusieurs nucléotides peuvent ainsi être éliminés et deux types d'insertion peuvent être trouvés.

L'addition de nucléotides de manière aléatoire résulte dans l'insertion de régions dites régions N sur les chaînes lourdes des immunoglobulines . Il a été émis l'hypothèse (Randau et al . , Molecular and Cellular Biology , 1987 , 3.237-3.243 ; que la désoxynucléotidyle transférase (TdT) est responsable de cette addition aléatoire de nucléotides .

Les insertions de nucléotides de type P correspondent à la répétition inverse des séquences adjacentes à celles des séquences codantes . On a émis l'hypothèse que leur addition correspond à une étape obligatoire du mécanisme de recombinaison .

Des expériences de transfection à l'aide d'ADN génomique ont permis d'isoler deux gènes intervenant de manière active dans la recombinaison : les gènes Rag-1 et Rag-2 (Oettinger et al., Science , Volume 248, 1517-1523, 1990).

Il a été montré que les gènes Rag-1 et Rag-2 sont responsables de la recombinaison site-spécifique.

Néanmoins, la combinaison des produits des gènes Rag-1 et Rag-2 ne permet pas de reconstituer la diversité des anticorps trouvés in vivo , c'est-à-dire ne permet pas de rajouter des séquences N .

Diverses méthodes ont été mises au point pour tenter de modifier les chaînes lourdes et légères des immunoglobulines ou des récepteurs.

Le brevet EP-368.684 concerne une méthode de clonage des séquences nucléotidiques correspondant aux domaines variables des molécules de la famille des immunoglobulines . Cette méthode consiste à fabriquer un ADN complémentaire du domaine variable de l'immunoglobuline .

Le brevet EP-328.444 quant à lui est relatif à une méthode de modification de la structure d'un anticorps tout en conservant sa spécificité fonctionnelle . Ainsi , des domaines constants notamment sont modifiés par une manipulation classique du génie génétique .

A la connaissance du demandeur , il n'existe pas de méthode permettant d'obtenir de manière efficace des immunoglobulines modifiées dans leurs structures , et ce avec une grande diversité dans les modifications.

Le demandeur s'est donc attaché à la mise en évidence des mécanismes de recombinaison qui permettent à l'organisme d'obtenir une grande diversité dans les immunoglobulines tels que les IgG, les IgM, les IgA, les IgE, et dans les récepteurs des cellules lymphoïdes.

Le demandeur s'est aussi attaché à la mise au point d'une méthode générale permettant d'obtenir de manière aléatoire une gamme très diversifiée de mutations , en particulier par insertion aléatoire, dans la séquence nucléotidique correspondant à des protéines, de structures et de fonctions diverses .

Le demandeur a ainsi montré de manière surprenante , que la combinaison des produits d'expression des gènes Rag-1 et Rag-2 permet d'obtenir une recombinaison site-spécifique et que l'introduction de la TdT entraîne une diversité de jonction entre les séquences DJ et VDJ équivalentes à celles trouvées in vivo.

Le demandeur a d'autre part mis en évidence que l'on pouvait en utilisant les produits de Rag-1 et Rag-2 ainsi que la terminale désoxynucléotidyle transférase, obtenir in vitro, la production d'anticorps présentant des réarrangements et qui statistiquement, présentent une grande diversité tant structurelle que fonctionnelle .

La présente invention a donc pour objet une composition contenant en combinaison dans des quantités synergiques les produits d'express ion des gènes Rag-1 et Rag-2 et une terminale désoxynucléotidyle transférase ou un ou plusieurs de leurs fragments biologiquement actifs .

Elle a d'autre part pour objet une composition contenant des séquences nucléotidiques préalablement purifiées portant les gènes Rag-1 et Rag-2 et le gène codant pour la TdT, dans laquelle avantageusement les séquences nucléotidiques sont portées par des vecteurs.

Elle est en outre relative à une composition comprenant les plasmides p Blue Rec (Kallenbach et al., Nucléic Acid Research (18, 6730, 1990), p Rag-1 et p Rag-2 (Oettinger et al, précédemment cité)
et pMTDT déposé auprès de la CNCM sous le n° I 1160 .

La présente invention a aussi pour objet :
- un procédé de génération d'une diversité structurale ou structurale et fonctionnelle dans une séquence peptidique par délétions ou insertions aléatoires de nucléotides dans une séquence nucléotidique codant pour cette séquence peptidique , ledit procédé comprenant la transfection d'une préparation cellulaire par un ou plusieurs vecteurs d'expression des produits des gènes Rag-1, Rag-2 et de la terminale désoxynucléotidyle transférase (TdT) et par un vecteur identique ou différent portant ladite séquence nucléotidique bordée de chaque côté par une ou plusieurs séquences de recombinaison RSS.
- procédé de génération d'une diversité structurale ou structurale et fonctionnelle dans une séquence peptidique par délétions ou insertions aléatoires de nucléotides dans une séquence nucléotidique codant pour cette séquence peptidique, ledit procédé comprenant la transfection d'une préparation cellulaire par un vecteur portant ladite séquence nucléotidique bordée de chaque côté par une ou plusieurs séquences de recombinaison RSS, puis dans une seconde étape par un ou plusieurs vecteurs identiques ou différents d' expression des produits des gènes Rag-1 , Rag-2 et de la terminale désoxynucléotidyle transférase.

De tels procédés permettent ainsi d'introduire des insertions et des délétions dans des séquences nucléotidiques, de manière aléatoire . Ces modifications de séquences permettent ainsi d'accroître à volonté la diversité biologique.

De telles méthodes sont notamment des substituts intéressants aux méthodes traditionnelles de mutagénèse et aux méthodes d'obtention d'anticorps monoclonaux par les hybridomes.

La possibilité d'obtenir des anticorps monoclonaux par une autre méthode que celle des hybridomes est avantageuse en thérapeutique humaine car les préparations d'anticorps obtenues selon l'invention sont pures.

On rappelle que l'on entend par séquences N des insertions aléatoires de nucléotides, c'est-à-dire qui ne sont pas des répliques de séquences préexistantes au voisinage des RSS.

Ces séquences N sont donc créées au hasard et de ce fait présentent une très grande diversité, qui n'est pas dépendante de la séquence nucléotidique au voisinage des RSS.

Les séquences P sont par contre des insertions résultant de la répétition inverse des séquences adjacentes aux RSS.

De ce fait , elles présentent une moins grande diversité que les régions N.

Avantageusement , le ou les vecteurs recombinés portant la séquence nucléotidique correspondant à la séquence peptidique sont transférés dans des bactéries afin de sélectionner les protéines ou les peptides présentant la structure et/ou la fonction souhaitée.

Il est à noter qu'il est nécessaire de choisir des vecteurs d'expression des protéines ou peptides adaptés aux cellules, eucaryotes ou procaryotes, utilisées . On peut ainsi utiliser les plasmides pcDNAI (commercialisé par In Vitrogen) ou pRc/CMV (commercialisé par In Vitrogen) pour les cellules eucaryotes ou le plasmide pBlue Script. (commercialisé par Stratagen).

La présente invention est d'autre part relative à une mise en oeuvre préférentielle du procédé pour l'obtention d'immunoglobulines, en particulier d'anticorps présentant une grande diversité structurelle et fonctionnelle par réarrangements séparés des chaînes légères et lourdes composant les immunoglobulines et co-expression dans une même cellule des deux chaînes.

Ainsi , ce mode de mise en oeuvre préférentiel permet d'obtenir une grande quantité de cellules exprimant diverses séquences des deux chaînes des immunoglobulines . Une étape ultérieure permet la sélection de l'immunoglobuline spécifique d'un agent pathogène donné par exemple.

Avantageusement, la séquence correspondant aux chaînes lourdes utilisées ne comprend que la partie Fab. de ces chaînes. La partie Fc est rajoutée ultérieurement.

De manière préférentielle , le réarrangement des chaînes légères est effectué en présence des séquences nucléotidiques des gènes Rag-1 et Rag-2 et le réarrangement des chaînes lourdes est effectué en présence des séquences nucléotidiques des gènes Rag-1 et Rag-2 et du gène de la terminale désoxynucléotidyle transférase.

Pour obtenir l'expression des chaînes lourdes des immunoglobulines en utilisera des vecteurs portant les segments V, D et J tandis que pour les chaînes légères les vecteurs porteront les séquences V et J.

La présente invention est en outre relative à un procédé pour l'obtention de récepteurs des cellules lymphoïdes, et en particulier des cellules T, présentant une grande diversité structurale et fonctionnelle par réarrangement des chaînes alpha, bêta, gamma et/ou delta des récepteurs des cellules T.

Elle a de plus pour objet un procédé, comprenant les étapes de :
a) transfection d'une préparation cellulaire par un ou plusieurs vecteurs portant une séquence nucléotidique codant pour la séquence peptidique dont on veut obtenir la variabilité et par un ou plusieurs vecteurs identiques ou différents d'expression des gènes Rag-1, Rag-2 et de la TdT;
b) isolement de l'ADN des vecteurs des préparations cellulaires ;
c) élimination des vecteurs n'ayant pas subi de recombinaison ;
d) transformation d'hôtes cellulaires par les vecteurs ayant subi une recombinaison résultant de l'étape c), et
e) sélection des hôtes cellulaires exprimant les molécules présentant la structure et/ou la fonction recherchée.

De manière avantageuse , ce procédé comprend les étapes de :
a) co-transfection d'une préparation cellulaire par un ou plusieurs vecteurs portant des gènes codant pour des chaînes légères non réarrangées et par un ou plusieurs vecteurs exprimant des gènes codant pour Rag-1 et Rag-2 , et
b) co-transfection d'une autre préparation cellulaire par un ou plusieurs vecteurs portant des gènes codant pour des chaînes lourdes non réarrangées et par un ou plusieurs vecteurs exprimant le gène de la terminale désoxynucléotidyle transférase ainsi que des gènes codant pour Rag-1 et Rag-2 ,
c) isolement de l'ADN des vecteurs des deux préparations cellulaires ,
d) élimination des vecteurs n'ayant pas subi de recombinaison ,
e) transformation d'au moins deux cultures bactériennes respectivement par les préparations de vecteurs obtenus à l'étape d), amplification et préparation des ADN des vecteurs bactériens ,
f) mise en place sur un même vecteur des gènes codants pour les chaînes lourdes et légères,
g) transformation d'hôtes cellulaires par le vecteur obtenu à l'étape f), et
h) sélection des hôtes cellulaires exprimant des molécules immunoglobulines complètes .

On appelle hôtes cellulaires toutes bactéries ou cellules eucaryotes pouvant être transformées ou transfectées.

On appelle, dans la présente demande , vecteur, toute molécule d'ADN autoréplicative et pouvant être transférée d'une cellule à une autre . On utilise préférentiellement des plasmides dans les étapes a à h mais tout autre vecteur compatible avec les systèmes cellulaires et bactériens employés peut être avantageusement utilisé .

Les vecteurs obtenus à l'étape c) et n'ayant pas subi de recombinaison sont avantageusement éliminés par digestion enzymatique à un site spécifiquement reconnu par une endonucléase de restriction .

Les cellules préférentiellement utilisées dans les étapes a) et b) sont des fibroblastes ou des cellules lymphoïdes, ou tout autre type cellulaire ou lignée de cellules eucaryotes .

Il est à noter que les vecteurs utilisés contiennent de préférence , la région précoce de polyome afin de permettre leur réplication à l'état autonome dans des cellules eucaryotes .

La sélection des bactéries à l'étape h) est avantageusement effectuée par réplique sur filtre des colonies bactériennes obtenues par étalement des bactéries sur boîtes de Pétri et criblage ultérieur [" Molecular cloning ; a Laboratory Manual " (Sambrok et al, Cold Spring Harbor Laboratory Press, New York, 1989)] avec des antigènes pour lesquels l'on veut obtenir des anticorps spécifiques .

On note de plus , que les vecteurs exprimant les anticorps ou les récepteurs des cellules lymphoïdes recherchés peuvent être modifiés ultérieurement de façon à permettre l'expression d'immunoglobulines complètes dans des cellules eucaryotes , et en particulier , de façon à permettre leur glycosylation .

Les modes de mise en oeuvre des étapes a) à h) sont à la portée de l'homme du métier . On peut de manière générale, se référer au " Molecular cloning; a Laboratory Manual" (Sambrok et al, Cold Spring Harbor Laboratory Press, New York , 1989)

Des mises de modes en oeuvre pratiques de ces étapes sont aussi décrites dans l'article de Huse et al. (Science , volume 246, 1275-1281, 1989).

Notamment , le vecteur utilisé dans l'étape a) peut être le plasmide p Blue Script contenant une cassette du type fragment EcoRI- NotI de lambda Lcl décrit dans cet article dans lequel sont insérés un segment V et un segment J fusionné à la région constante de la chaîne légère. Les segments VL et JL sont bordés par leurs séquences signal de recombinaison.

Le vecteur utilisé pour la co-transformation de l'étape b) peut être le plasmide p Blue Script contenant une cassette du type fragment NotI-EcoRI de lambda Hc2 décrit dans cet article dans lequel sont insérés un segment V, un segment D et un segment J fusionné à la région CH1 de la chaîne lourde. Les segments VH, DH, et JH sont bordés par leurs séquences signal de recombinaison.

Les vecteurs d'expression des gènes Rag-1 et Rag-2 utilisés dans les étapes a) et b) peuvent être ceux décrits par Oettinger et al. (Science, 248, 1517-1523, 1990).

Le vecteur de clonage portant le gène codant pour la terminale désoxynucléotidyle transférase peut être en particulier le plasmide pMTdT qui est un pcDNAII dans lequel a été inséré l'ADN complémentaire de la terminale désoxyribonucléotide transférase de souris .

Ce plasmide qui a été déposé le 10 Décembre 1991 auprès de la Collection Nationale de Culture des Microorganismes de l'Institut Pasteur sous le n° I 1160 est un autre objet de la présente demande.

L'article de Huse et al. précédemment cité mentionne d'autre part plus particulièrement des modes de mise en oeuvre pratiques qui peuvent être utilisés dans le cadre de la présente invention .

En particulier , les vecteurs utilisés dans les étapes a) et b) peuvent être obtenus à partir d'une librairie construite comme décrit dans cet article .

Cette librairie peut être obtenue en clonant les fragments des chaînes légères et lourdes dans respectivement les vecteurs issus du phage lambda , lambda Lc1 et lambda Hc2. Ces vecteurs qui servent au clonage dans l'étape initiale de la construction de la librairie peuvent être excisés et donner naissance à un plasmide contenant des morceaux d'oligonucléotides correspondant aux chaînes lourdes et légères.

Ces vecteurs contiennent divers sites de restriction, et une séquence codante pour le peptide leader du gène bactérien PelB qui a été précédemment utilisé avec succès dans E. coli pour secréter des fragments Fab , un site de liaison des ribosomes , et sur le vecteur lambda Hc2 une séquence correspondant au décapeptide tag situé à l'extrémité C terminale de l'insertion de la chaîne lourde. Le peptide tag permet la purification des produits d'expression par passage sur des colonnes d'immuno-affinite .

L'ADN à la base de la fabrication de la librairie des chaînes lourdes est préférentiellement de l'ADN humain afin de minimiser les risques de rejet par l'organisme dans le cas d'utilisation de ces anticorps en thérapeutique humaine.

La mise en oeuvre de l'étape f), qui est la mise en place sur un même vecteur des gènes codant pour les chaînes lourdes et légères peut-être effectuée comme décrite page 1278 de l'article de Huse et al. précédemment cité .

On digère ainsi la librairie de chaînes légères par l'endonucléase de restriction coupant à un site unique , on déphosphoryle les extrémités 5' résultantes , et on digère ensuite les produits , par une autre endonucléase de restriction Eco R1 coupant à un site unique .

L'ADN des vecteurs composant la librairie des chaînes lourdes est clivé par l'endonucléase Hind-III puis déphosphorylé et digéré par l'endonucléase Eco R1.

Les ADN ainsi préparés sont mélangés et réunis par ligation .

Après ligation , seuls les clones qui résultent de la combinaison d'un fragment issu de la librairie de chaînes lourdes et d'un fragment issu de la librairie de chaînes légères, reconstituent un phage viable.

Pour la construction des librairies des chaînes lourdes et légères, on peut utiliser des préparations d'ADN messagers isolées à partir de cellules de l'organisme ou d'hybridomes. On synthétise alors dans un système d'amplification par PCR des ADN complémentaires correspondants. Ces techniques sont bien connues de l'homme du métier et sont notamment décrites dans " Molecular cloning a Laboratory Manual (Sambrok et al. 1989 , précédemment cité).

La sélection des bactéries exprimant les molécules complètes de l'étape h) est suivie par une étape de sélection des clones synthétisant les molécules que l'on souhaite obtenir.

L'assemblage des parties des chaînes lourdes et des chaînes légères ainsi obtenu peut conduire uniquement à l'obtention du fragment Fab. Le vecteur est alors modifié de façon à pouvoir coder pour le fragment Fc . Le produit d'expression de ce vecteur est alors un anticorps.

Dans le cas de la sélection de clones synthétisant des anticorps , une méthode de sélection peut être celle utilisée par Huse et al. (précédemment cité) pour la sélection de clones synthétisant des anticorps dirigés à l'encontre du paranitrophényle phosphonamidate (NPN) .

La méthode utilisée dans cet article consiste à faire des doubles sur feuille de nitrocellulose de clones étalés sur des boîtes de milieu de culture gélifié et de tester l'hybridation du NPN couplé à du sérum d'albumine bovine marqué à l'¹²⁵I.

Les produits d'expression dans des cellules eucaryotes ou procaryotes transfectées par des plasmides recombinants portant des gènes originaires du lapin ou de la souris peuvent être utilisés dans des coffrets de diagnostic humain ou vétérinaire.

La présente invention est illustrée sans pour autant être limitée par les exemples de mise en oeuvre suivants dans lesquels :
La figure 1 représente les séquences des jonctions formées sur le plasmide pBlueRec après co-transfection avec pRag-1 et p Rag-2 dans des fibroblastes NIH-3T3 .
La figure 2 représente les séquences des jonctions formées sur le plasmide pBlueRec après co-transfection avec des vecteurs codant pour Rag-1 , Rag-2 et la TdT dans des fibroblastes NIH-3T3.
La figure 3 représente les séquences de jonctions formées sur pBlueRec après co-transfection avec des vecteurs codant pour Rag-1 et Rag-2 dans les lignées cellulaires BW1J, CHO-K1 et A.9

Sur ces trois figures , les séquences des plasmides recombinés sont alignées avec la séquence du plasmide d'origine pBlueRec qui est la première séquence en partant du haut des figures ..

Sur ces trois figures , les nucléotides supposés être dus à des insertions de type P sont soulignés dans les parties centrales des figures , tandis que les insertions de type N figurées dans ces mêmes parties ne sont pas soulignées. Les délétions sont représentées par des lignes discontinues. EXEMPLES

### Matériels et méthodes utilisés dans les exemples .

### Lignée cellulaire

Les fibroblastes d'embryons de souris NIH-3T3 (ATCC CRL 6442) et les cellules A9 dérivées des cellules L , (ATCC CRL. 6319) sont cultivés dans du DMEM complémenté avec 10% de sérum de foetus de veau.

Les cellules d'hépatomes de souris BW1J sont cultivées comme indiqué par Cassio D. & Weiss M.C. (Somat Cell Genet, 5, 719-738, 1979) .

Les cellules d'ovaires d'hamster chinois CHO-K1 (ATCC CCL 61) sont cultivées dans du RPMI , complémentées avec 10% de sérum de foetus de veau .

Les cellules pré-B BASP-1 (Choquet et al.,Science, 235, 1211-1214, 1987) sont cultivées dans du RPMI complémenté avec 10% de sérum de foetus de veau et 50 *µ*m de *β*-mercapto- éthanol.

### Clonage du gène codant pour la terminale désoxynucléotidyle transférase de souris.

De l'ARN est préparé à partir de thymus de souris vieilles de 5 semaines comme décrit par Auffray et Rougeon (Europe J. Biochem., 107, 303-314, 980), du thiocyanate de guanidine 4M étant utilisé à la place d'urée 6M.

L'ARN poly-A est purifié en utilisant une chromatographie cellulose oligo DT et est analysé par Northern blot . La synthèse du simple brin est effectuée à partir de 5 *µ*g d'ARN poly-A initialisé avec de l'oligo DT en utilisant la transcriptase inverse du MMLV (commercialisée par BRL).

La synthèse du double brin est effectuée en présence d'ADN polymérase I et de RNase H .

Des adaptateurs double brin portant des extrémités BstX1 sont liés au CDNA préparé de manière adéquate et clonés dans le site de restriction BstX1 du pCDNA2 (commercialisé par In Nitrogen).

La librairie d'ADN complémentaire de thymus de souris est criblée avec deux oligo-nucléotides mélangés correspondent respectivement aux séquences 121-142 et 1471-1494 de la séquence de l'ADN complémentaire de la TdT de souris .

Les clones d'ADN complémentaire positifs et que l'on suppose suffisamment longs pour contenir entièrement le gène codant pour la TdT de souris sont séquencés sur les deux brins par la méthode de la didésoxyterminaison (Sanger et al., PNAS, 74, 5463-5467, 1977) .

### Vecteurs utilisés.

Le plasmide pBlueRec est décrit par Kallenbach et al.((1990) Nucleic Acid Research 18 , 6730).

p Rag-1 et p Rag-2 ont été fournis par Oettinger et al. (précédemment cité).

L'ADN complémentaire de la TdT de souris est cloné dans le pCDNA1. L'expression de Rag-1 , Rag-2 et de la TdT sont sous le contrôle du promoteur du CMV.

### Mise en évidence de la recombinaison spécifique

Les transfections sont effectuées par électroporation en suivant les indications données par Chu et al .(Nucleic Acid Research Res., 15, 1311-1326, 1987).

2.10⁶ cellules sont transfectées avec 2,5 µg de pBlueRec avec ou sans 6 µg de p Rag-1 ou 4,8 *µ*g de p Rag-2.

Pour déterminer l'effet de la TdT sur l'insertion de région N , 4,5 µ g du vecteur d'expression de la TdT sont ajoutés aux trois vecteurs mentionnés ci-dessus .

Les cellules sont récoltées après 40 à 48 heures d'incubation à 37°C , lavées avec du PBS et l'ADN plasmidique est préparé selon Birnboim et Doly (Nucleic Acids Res. 7, 1513-1523, 1979).

Les culots d'ADN sont resuspendus dans 20 *µ*l d'eau stérile . 7 *µ*l de la solution d'ADN sont digérés par Dpnl afin d'éliminer les plasmides qui ne sont pas répliqués.

40 *µ*l d'une bactérie compétente XL1-Blue (commercialisée par Stratagène) sont transformés par électroporation et étalés sur des boîtes d'Agar LB contenant du XGal (80 *µ*g/ml), de l'IPTG (150 *µ*M) de l'ampicilline (100 *µ*g/ml) et de la tétracycline (10 *µ*g/ml).

La fréquence de réarrangement est calculée comme la quantité de colonies bleues x 3 sur le nombre total de clones.

### Séquençage des clones recombinants

Les colonies bleues sont repiquées et isolées sur des boîtes d'agar LB contenant du XGal , de l'IPTG, de l'ampicilline et de la tétracycline .

Les préparations d'ADN sont effectuées selon la méthode décrite par Sambrok et al.. (Molecular cloning, Laboratory Manual précédemment cité), puis traitées par de la RNase durant deux heures à température ambiante avant d'effectuer le séquençage du double brin .

### EXEMPLE 1

### Comparaison des fréquences de recombinaison dans les fibroblastes NIH-3T3 et dans les lignées cellulaires précurseurs des cellules B en présence de Rag-1 et Rag-2.

L'activité recombinatoire due à Rag-1 et Rag-2 dans les fibroblastes NIH-3T3 a été testée par transfection transitoire.

p Rag-1 et p Rag-2 sont co-transfectés dans des fibroblastes NIH3 avec le plasmide substrat pour la recombinaison pBlueRec.

Après 48 heures , l'ADN plasmidique est isolé et testé chez E.Coli pour la recombinaison .

La séquence LacZ de pBlueRec est interrompue par un fragment d'ADN de 280 paires de bases flanquée de deux RSS.

Le réarrangement site-spécifique va éliminer l'insertion et dans un cas sur trois va restaurer, le cadre de lecture correcte , donnant naissance à des clones bleus après transformation de E.Coli.

Ce test rapide permet d'examiner un nombre important de réarrangements .

Des expériences de transfection avec p Rag-1 ou p Rag-2 seuls ne donnent pas naissance à des clones recombinants.

Comme indiqué sur le tableau I , on observe une fréquence de recombinaison importante quand les deux plasmides sont co-transfectés . De plus , la fréquence (moyenne géométrique= 1,26) est comparable à celle observée après transfection du substrat de recombinaison dans les cellules pré B , BASP1 (moyenne géométrique = 1,46).

Afin de comparer les jonctions codantes formées après la recombinaison modulée par p Rag-1 et p Rag-2 dans les fibroblastes , avec les jonctions observées dans les cellules lymphoïdes, les jonctions sur les plasmides réarrangés obtenues après transfection des cellules NIH-3T3 sont séquencées .

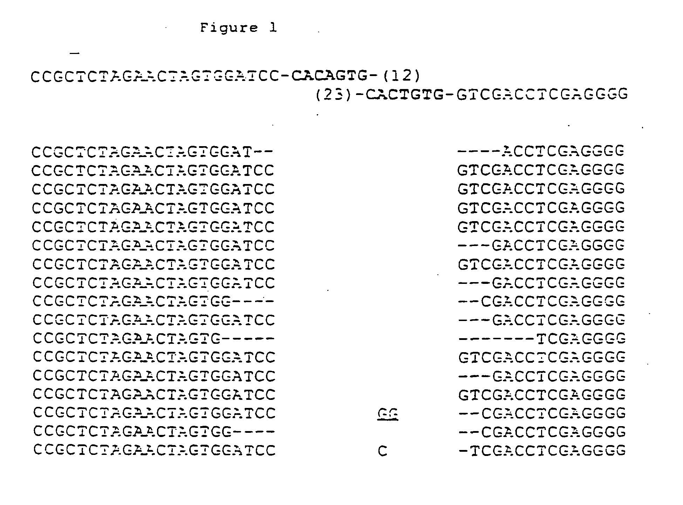

Les séquences indiquées sur la figure 1 représentent des éléments de recombinaison indépendants , c'est-à-dire issus de différentes expériences de transfection .

Sept jonctions sur 17 présentent ni délétions ni insertions .

Quatre jonctions ont des délétions sur un côté et quatre autres ont des délétions sur les deux côtés.

Seulement une jonction présente une insertion de type P de deux paires de bases associée à une délétion de deux paires de bases sur un des côtés de la jonction .

La dernière jonction présente une délétion d'une paire de base et une addition d'un nucléotide , qui peut être attribuée à l'heptamère et est probablement due à une excision imprécise.

### EXEMPLE 2

### Co-transfection par Rag-1 et Rag-2 et TdT des fibroblastes NIH-3T3.

Afin de tenter de reconstituer, la diversité jonctionnelle observée dans des cellules préB ou préT, des fibroblastes NIH-3T3 sont transfectés avec le vecteur d'expression de la TdT ainsi qu'avec p Rag-1, p Rag-2 et pBlueRec.

Les plasmides recombinants sont séquencés.

Des transfections témoins effectuées avec le vecteur pCDNAl sans l'ADN complémentaire de la TdT n'entraîne aucune insertion de régions N .

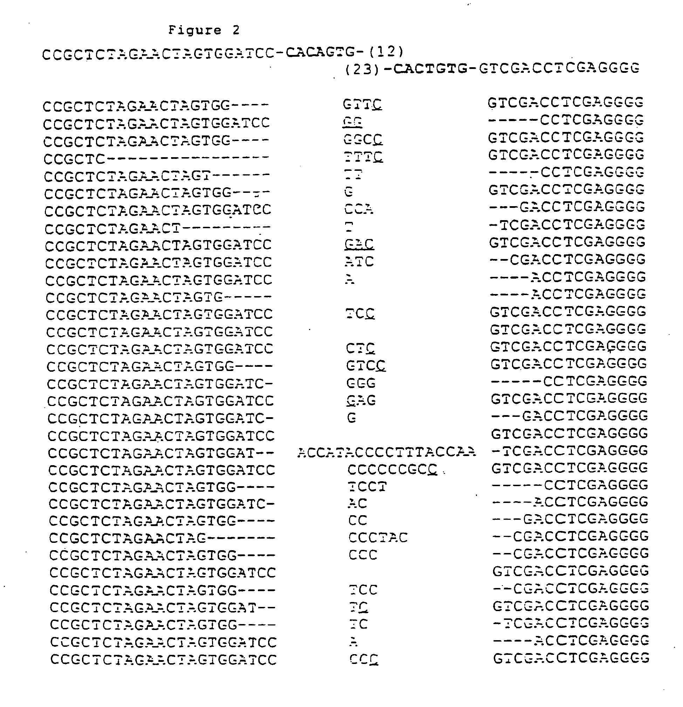

Comme l'indique la figure 2, 88 % des jonctions montrent qu'il y a eu insertion de régions N . La plupart des régions N sont de 1 à 4 nucléotides avec une moyenne de 3 nucléotiques par jonction

On observe néanmoins une insertion exceptionnelle de 18 nucléotides .

La TdT incorpore plus efficacement les résidus G que d'autres nucléotides . La fréquence des insertions de nucléotides de type P semble augmenter dans cette expérience. Il doit cependant être remarqué qu'il est impossible de les distinguer des régions N.

### EXEMPLE 3

### Effet de Raq-1 et Rag-2 dans différents types de lignées cellulaires différenciées.

On a montré dans les exemples précédents , que Rag-1 et Rag-2 sont capables d'entraîner une activité de recombinaison dans des cellules relativement indifférenciées que sont les fibroblastes NIH-3T3.

On a donc testé l'activité de ces deux gènes dans des cellules dans des états différenciés . Les résultats obtenus dans les lignées cellulaires BW1J , CHO-K1 et A9 sont indiqués sur le tableau 2.

On observe des variations entre les différentes lignées cellulaires, mais de manière surprenante, les fréquences de réarrangement dans des lignées BW1J et CHO-K1 sont clairement supérieures à celles obtenues pour les fibroblastes 3T3.

Les réarrangements peuvent être détectés 13 heures après la co-transfection avec pBlueRec , p Rag-1 et p Rag-2.

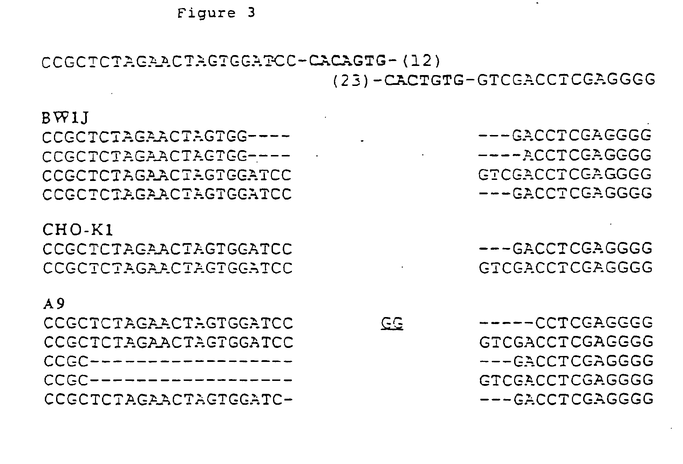

Le séquençage des plasmides recombinés montre que des délétions aux jonctions codantes ont lieu dans les trois lignées cellulaires testées (figure 3).

Une seule insertion de nucléotide de type P est trouvée à la jonction d'un clone recombinant obtenu après transfection des lignées A9.

### CONCLUSION.

L'ensemble de ces résultats montre qu'on obtient dans des lignées non différenciées des délétions nucléotidiques après co-transfection par p Rag-1 et p Rag-2. On observe de plus des insertions nucléotidiques de type P telles que définies par Lafaille et al. (Cell, 59, 859-870, 1989) .

On remarque de plus que la co-expression de Rag-1, Rag-2 et TdT dans des cellules non différenciées conduit à des insertions de type N.

L'ensemble de ces résultats indique donc que Rag-1 et Rag-2 suffisent à induire une recombinaison site-spécifique mais que la présence de la TdT , en combinaison avec Rag-1 et Rag-2, est nécessaire pour obtenir des insertions de type N qui sont le reflet de l'expression de la diversité de synthèse des immunoglobulines et des récepteurs des cellules lymphoïdes.

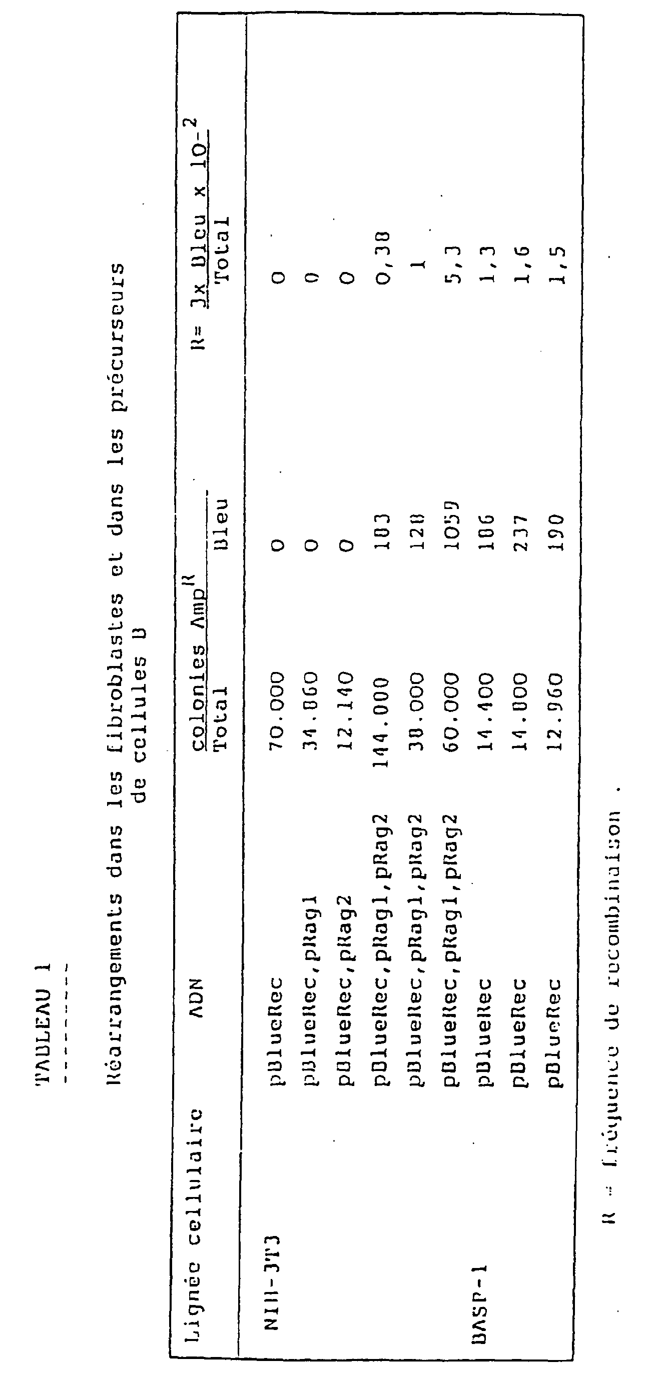

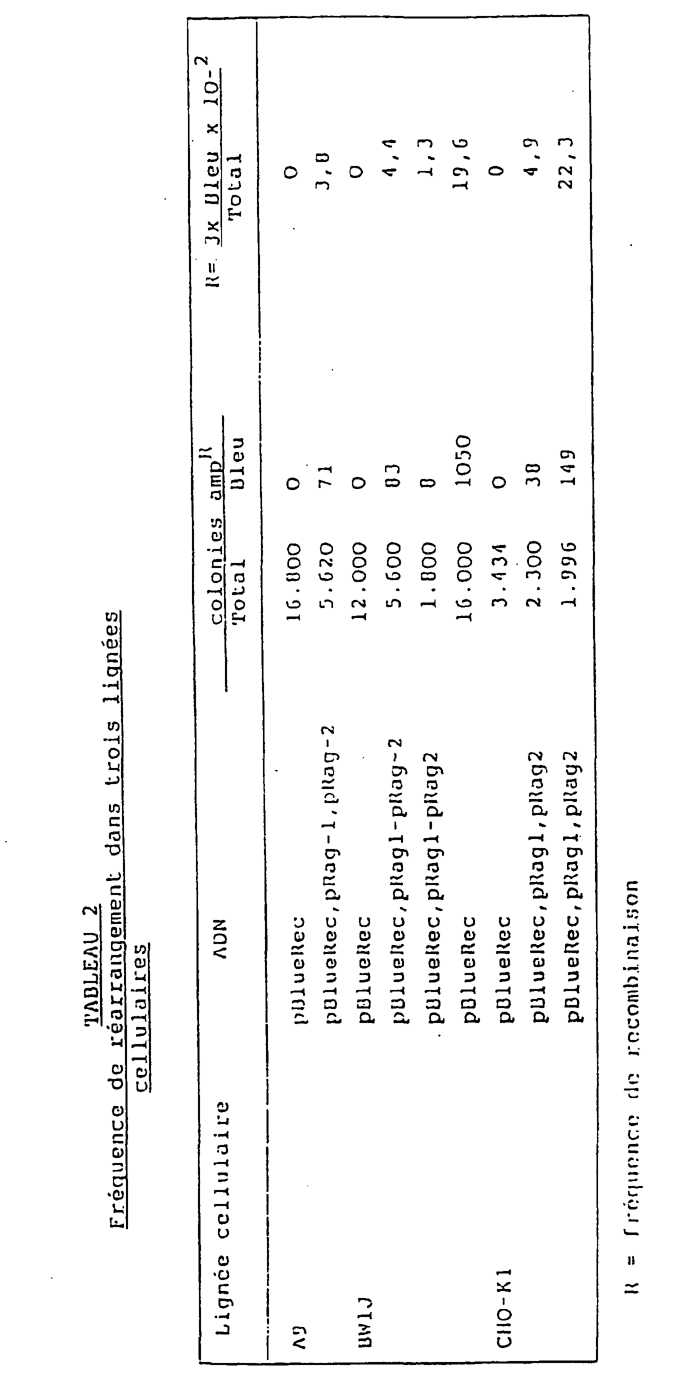

## Revendications

1. Composition contenant des séquences nucléotidiques préalablement purifiées portant les gènes Rag-1 et Rag-2 et le gène codant pour la TdT.

2. Composition selon la revendication 1, **caractérisée en ce que** les séquences nucléotidiques préalablement purifiées sont portées sur des vecteurs.

3. Composition comprenant les plasmides pBlueRec, p Rag-1, p Rag-2 et pMTDT déposé auprès de la CNCM sous le n°I-1160.

4. Composition contenant en combinaison les produits d'expression préalablement purifiés des gènes Rag-1 et Rag-2 et la terminale désoxynucléotidyle transférase.

5. Procédé de génération d'une diversité structurale ou structurale et fonctionnelle dans une séquence peptidique par délétions ou insertions aléatoires de nucléotides dans une séquence nucléotidique codant pour cette séquence peptidique, ledit procédé comprenant la transfection d'une préparation cellulaire par un ou plusieurs vecteurs d'expression des produits des gènes Rag-1, Rag-2 et de la terminale désoxynucléotidyle transférase (TdT) et par un vecteur identique ou différent portant ladite séquence nucléotidique bordée de chaque côté par une ou plusieurs séquences de recombinaison RSS.

6. Procédé de génération d'une diversité structurale ou structurale et fonctionnelle dans une séquence peptidique par délétions ou insertions aléatoires de nucléotides dans une séquence nucléotidique codant pour cette séquence peptidique, ledit procédé comprenant la transfection d'une préparation cellulaire par un vecteur portant ladite séquence nucléotidique bordée de chaque côté par une ou plusieurs séquences de recombinaison RSS, puis dans une seconde étape par un ou plusieurs vecteurs identiques ou différents d'expression des produits des gènes Rag-1, Rag-2 et de la terminale désoxynucléotidyle transférase.

7. Procédé selon l'une des revendications 5 à 6, **caractérisé en ce que** le ou les vecteurs recombinés portant la séquence nucléotidique correspondant à la séquence peptidique sont transférés dans des bactéries afin de sélectionner les protéines présentant la structure et/ou la fonction souhaitée.

8. Procédé selon l'une des revendications 5 à 7, pour l'obtention d'immunoglobulines et notamment d'anticorps présentant une grande diversité structurelle et fonctionnelle par réarrangement séparé des chaînes légères et lourdes et co-expression des deux chaînes.

9. Procédé selon l'une des revendications 5 à 7, pour l'obtention de récepteurs des cellules lymphoïdes, et en particulier des cellules T, présentant une grande diversité structurale et fonctionnelle par réarrangement des chaînes alpha, bêta, gamma et/ou delta des récepteurs des cellules T.

10. Procédé selon la revendication 8, **caractérisé en ce que** le réarrangement des chaînes lourdes est effectué en présence des séquences nucléotidiques des gènes Rag-1 , Rag-2, et du gène de la terminale désoxynucléotidyle transférase, lesdites séquences étant effectivement exprimées.

11. Procédé selon l'une des revendications 5 et 6, comprenant les étapes de :
a) transfection d'une préparation cellulaire par un ou plusieurs vecteurs portant une séquence nucléotidique codant pour la séquence peptidique dont on veut obtenir la variabilité et par un ou plusieurs vecteurs identiques ou différents d'expression des gènes Rag-1, Rag-2 et de la TdT ;
b) isolement de l'ADN des vecteurs des préparations cellulaires ;
c) élimination des vecteurs n'ayant pas subi de recombinaison ;
d) transformation d'hôtes cellulaires par les vecteurs ayant subi une recombinaison résultant de l'étape c), et
e) sélection des hôtes cellulaires exprimant les molécules présentant la structure et/ou la fonction recherchée.

12. Procédé selon la revendication 11, **caractérisé en ce que** ladite séquence peptidique est une chaîne alpha, bêta, delta, ou gamma des récepteurs des cellules lymphoïdes ou un de leurs fragments ou un de leurs dérivés.

13. Procédé selon l'une des revendications 8 et 10, comprenant les étapes de :
a) co-transfection d'une préparation cellulaire par un ou plusieurs vecteurs portant des séquences nucléotidiques codant pour des chaînes légères non-réarrangées et par un ou plusieurs vecteurs exprimant les gènes Rag-1 et Rag-2, et
b) co-transfection d'une autre préparation cellulaire par un ou plusieurs vecteurs portant des séquences nucléotidiques codant pour des chaînes lourdes non-réarrangées et par un ou plusieurs vecteurs exprimant le gène de la terminale désoxynucléotidyle transférase ainsi que les gènes Rag-1 et Rag-2 ;
c) isolement de l'ADN des vecteurs des deux préparations cellulaires ;
d) élimination des vecteurs n'ayant pas subi de recombinaison,
e) transformation d'au moins deux cultures bactériennes respectivement par les deux préparations obtenues à l'étape d), amplification et préparation des ADN des vecteurs bactériens,
f) mise en place sur un même vecteur des gènes codant pour les chaînes lourdes et légères,
g) transformation d'hôtes cellulaires par le vecteur obtenu à l'étape f), et
h) sélection des hôtes cellulaires exprimant des molécules d'immunoglobulines complètes.

14. Procédé selon la revendication 13, **caractérisé en ce que** les vecteurs obtenus à l'étape c) et n'ayant pas subi de recombinaison sont éliminés par digestion enzymatique.

15. Procédé selon la revendication 13, **caractérisé en ce que** les préparations cellulaires sont des fibroblastes ou toute autre cellule eucaryote.

16. Procédé selon la revendication 13, **caractérisé en ce que** le vecteur exprimant la terminale désoxyribonucléotidyle transférase est le plasmide pMTdT portant le gène de la terminale désoxyribonucléotide transférase déposé auprès de la Collection Nationale de Cultures de Microorganismes sous le N°I-1160.

## Claims

1. A composition containing previously purified nucleotide sequences carrying the Rag-1 and Rag-2 genes and the gene encoding the TdT.

2. A composition according to claim 1, **characterized in that** the previously purified nucleotide sequences are carried on vectors.

3. A composition comprising the pBlueRec, p Rag-1, p Rag-2 and pMTDT plasmids deposited with the CNCM under the no. I-1160.

4. A composition containing in combination the previously purified expression products of the Rag-1 and Rag-2 genes and the terminal deoxynucleotidyl transferase.

5. A method for generating a structural or structural and functional diversity in a peptide sequence by random deletions or insertions of nucleotides in a nucleotide sequence encoding this peptide sequence, said method comprising the transfection of a cell preparation by one or more expression vectors of the Rag-1, Rag-2 gene products and the terminal deoxynucleotidyl transferase (TdT) and by an identical or different vector carrying said nucleotide sequence flanked on either side by one or more RSS recombination sequences.

6. A method for generating a structural or structural and functional diversity in a peptide sequence by random deletions or insertions of nucleotides in a nucleotide sequence encoding this peptide sequence, said method comprising the transfection of a cell preparation by a vector carrying said nucleotide sequence flanked on either side by one or more RSS recombination sequences, and then in a second step, by one or more identical or different vectors for expressing Rag-1, Rag-2 gene products and the terminal deoxynucleotidyl transferase.

7. A method according to one of claims 5 to 6, **characterized in that** the recombinant vector(s) carrying the nucleotide sequence corresponding to the peptide sequence are transferred in bacteria in order to select the proteins having the desired structure and/or function.

8. A method according to one of claims 5 to 7 for obtaining immunoglobulins and notably antibodies having a high structural and functional diversity by separated rearrangement of the light and heavy chains and co-expression of both chains.

9. A method according to one of claims 5 to 7 for obtaining lymphoid cell and particularly T cell receptors having a high structural and functional diversity by rearranging the alpha, beta, gamma and/or delta chains of the T cell receptors.

10. A method according to claim 8, **characterized in that** the rearrangement of the heavy chains is carried out in the presence of the nucleotide sequences of Rag-1, Rag-2 genes and the terminal deoxynucleotidyl transferase gene, said sequences being actually expressed.

11. A method according to one of claims 5 and 6, comprising the steps of:
a) transfecting a cell preparation by one or more vectors carrying a nucleotide sequence encoding the peptide sequence the variability of which is wanted to obtain and by one or more identical or different vectors for expressing the Rag-1, Rag-2 and TdT genes;
b) isolating the DNA of the cell preparation vectors;
c) removing the vectors which have not undergone any recombination;
d) transforming cell hosts by the vectors which have undergone a recombination resulting from step c), and
e) selecting the cell hosts expressing the molecules with the desired structure and/or function.

12. A method according to claim 11, **characterized in that** said peptide sequence is an alpha, beta, delta or gamma chain of the lymphoid cell receptors or a fragment thereof or a derivative thereof.

13. A method according to one of claims 8 and 10, comprising the steps of:
a) co-transfecting a cell preparation by one or more vectors carrying nucleotide sequences encoding unrearranged light chains and by one or more vectors expressing the Rag-1 and Rag-2 genes, and
b) co-transfecting another cell preparation by one or more vectors carrying nucleotide sequences encoding unrearranged heavy chains and by one or more vectors expressing the terminal deoxynucleotidyl transferase gene as well as the Rag-1 and Rag-2 genes;
c) isolating the vector DNA from both cell preparations;
d) removing the vectors which have not undergone any recombination,
e) transforming at least two bacterial cultures respectively by the two preparations obtained in step d), amplifying and preparing the DNAs of the bacterial vectors,
f) placing on the same vector genes encoding the heavy and light chains,
g) transforming cell hosts by the vector obtained in step f), and
h) selecting the cell hosts expressing complete immunoglobulin molecules.

14. A method according to claim 13, **characterized in that** the vectors obtained in step c) and which have not undergone any recombination are removed by enzymatic digestion.

15. A method according to claim 13, **characterized in that** the cell preparations are fibroblasts or any other eukaryotic cell.

16. A method according to claim 13, **characterized in that** the vector expressing the terminal deoxyribonucleotidyl transferase is the pMTdT plasmid carrying the terminal deoxyribonucleotide transferase gene deposited with the Collection Nationale de Cultures de Microorganismes under the no. I-1160.

## Patentansprüche

1. Zusammensetzung, die vorgereinigte Nucleotidsequenzen enthält, welche die Gene Rag-1 und Rag-2 sowie das für TdT codierende Gen tragen.

2. Zusammensetzung gemäß Anspruch 1, **dadurch gekennzeichnet, dass** die vorgereinigten Nucleotidsequenzen von Vektoren getragen werden.

3. Zusammensetzung, die die Plasmide pBlueRec, pRag-1, pRag-2 und pMTDT, das bei der CNCM unter der Nr. I-1160 hinterlegt ist, umfasst.

4. Zusammensetzung, die eine Kombination der vorgereinigten Expressionsprodukte der Gene Rag-1 und Rag-2 und der terminalen Desoxynucleotidyl-Transferase enthält.

5. Verfahren zur Herstellung einer strukturellen oder strukturellen und funktionellen Vielfalt in einer Peptidsequenz durch zufällige Deletionen oder Insertionen von Nucleotiden in einer Nucleotidsequenz, die für diese Peptidsequenz codiert, wobei das Verfahren die Transfektion eines Zellpräparats mit einem oder mehreren Expressionsvektoren der Produkte der Gene Rag-1 und Rag-2 und der terminalen Desoxynucleotidyl-Transferase (TdT) und mit einem gleichen oder davon verschiedenen Vektor, der die auf beiden Seiten von einer oder mehreren Rekombinationssignalsequenzen (RSS) flankierte Nucleotidsequenz trägt, umfasst.

6. Verfahren zur Herstellung einer strukturellen oder strukturellen und funktionellen Vielfalt in einer Peptidsequenz durch zufällige Deletionen oder Insertionen von Nucleotiden in einer Nucleotidsequenz, die für diese Peptidsequenz codiert, wobei das Verfahren die Transfektion eines Zellpräparats mit einem Vektor, der die auf beiden Seiten von einer oder mehreren Rekombinationssignalsequenzen (RSS) flankierte Nucleotidsequenz trägt, und dann in einem zweiten Schritt mit einem oder mehreren gleichen oder davon verschiedenen Expressionsvektoren der Produkte der Gene Rag-1, Rag-2 und der terminalen Desoxynucleotidyl-Transferase umfasst.

7. Verfahren gemäß einem der Ansprüche 5 bis 6, **dadurch gekennzeichnet, dass** der bzw. die rekombinanten Vektoren, die die der Peptidsequenz entsprechende Nucleotidsequenz tragen, in Bakterien übertragen werden, um die Proteine, die die gewünschte Struktur und/oder Funktion aufweisen, zu selektieren.

8. Verfahren gemäß einem der Ansprüche 5 bis 7 zur Gewinnung von Immunglobulinen und insbesondere Antikörpern, die eine große strukturelle und funktionelle Vielfalt aufweisen, durch getrennte Umlagerung der leichten und schweren Ketten und Coexpression der beiden Ketten.

9. Verfahren gemäß einem der Ansprüche 5 bis 7 zur Gewinnung von Rezeptoren von lymphoiden Zellen und insbesondere T-Zellen, die eine große strukturelle und funktionelle Vielfalt aufweisen, durch Umlagerung der α-, β-, γ- und/oder δ-Ketten der T-Zell-Rezeptoren.

10. Verfahren gemäß Anspruch 8, **dadurch gekennzeichnet, dass** die Umlagerung der schweren Ketten in Gegenwart der Nucleotidsequenzen der Gene Rag-1 und Rag-2 und des Gens der terminalen Desoxynucleotidyl-Transferase erfolgt, wobei die Sequenzen effektiv exprimiert werden.

11. Verfahren gemäß einem der Ansprüche 5 und 6, das die folgenden Schritte umfasst:
a) Transfektion eines Zellpräparats mit einem oder mehreren Vektoren, die eine Nucleotidsequenz tragen, die für die Peptidsequenz codiert, bei der man eine Vielfalt erhalten möchte, und mit einem oder mehreren gleichen oder davon verschiedenen Expressionsvektoren der Gene Rag-1, Rag-2 und der TdT;
b) Isolierung der DNA der Vektoren aus den Zellpräparaten;
c) Beseitigung der Vektoren, die keine Rekombination erfahren haben;
d) Transformation von Wirtszellen mit Vektoren, die eine Rekombination erfahren haben, aus Schritt c); und
e) Selektion der Wirtszellen, die die Moleküle exprimieren, welche die gewünschte Struktur und/oder Funktion aufweisen.

12. Verfahren gemäß Anspruch 11, **dadurch gekennzeichnet, dass** es sich bei der Peptidsequenz um eine α-, β-, δ- oder γ-Kette der Rezeptoren von lymphoiden Zellen oder um eines ihrer Fragmente oder eines ihrer Derivate handelt.

13. Verfahren gemäß einem der Ansprüche 8 und 10, das die folgenden Schritte umfasst:
a) Cotransfektion eines Zellpräparats mit einem oder mehreren Vektoren, die Nucleotidsequenzen tragen, die für nicht umgelagerte leichte Ketten codieren, und mit einem oder mehreren Vektoren, die die Gene Rag-1 und Rag-2 exprimieren; und
b) Cotransfektion eines anderen Zellpräparats mit einem oder mehreren Vektoren, die Nucleotidsequenzen tragen, die für nicht umgelagerte schwere Ketten codieren, und mit einem oder mehreren Vektoren, die das Gen der terminalen Desoxynucleotidyl-Transferase sowie die Gene Rag-1 und Rag-2 exprimieren;
c) Isolierung der DNA der Vektoren aus den beiden Zellpräparaten;
d) Beseitigung der Vektoren, die keine Rekombination erfahren haben;
e) Transformation von wenigstens zwei Bakterienkulturen mit den beiden in Schritt d) erhaltenen Präparaten, Amplifikation und Präparation der DNA der bakteriellen Vektoren;
f) Anordnen der Gene, die für die schweren und leichten Ketten codieren, auf demselben Vektor;
g) Transformation von Wirtszellen mit dem in Schritt f) erhaltenen Vektor; und
h) Selektion der Wirtszellen, die die Moleküle von vollständigen Immunglobulinen exprimieren.

14. Verfahren gemäß Anspruch 13, **dadurch gekennzeichnet, dass** die in Schritt c) erhaltenen Vektoren, die keine Rekombination erfahren haben, durch enzymatischen Abbau beseitigt werden.

15. Verfahren gemäß Anspruch 13, **dadurch gekennzeichnet, dass** es sich bei den Zellpräparaten um Fibroblasten oder irgendwelche anderen eukaryontischen Zellen handelt.

16. Verfahren gemäß Anspruch 13, **dadurch gekennzeichnet, dass** es sich bei dem Vektor, der die terminale Desoxynucleotidyl-Transferase exprimiert, um das Plasmid pMTdT handelt, der das Gen der terminalen Desoxynucleotidyl-Transferase trägt und bei der Collection Nationale de Cultures de Microorganismes unter der Nr. I-1160 hinterlegt ist.
